# EUROPEAN PATENT SPECIFICATION

(11) **EP 0 407 357 B1**
(45) Date of publication and mention of the grant of the patent: **14.09.1994**
(21) Application number: 90830288.8
(22) Date of filing: 25.06.1990
(51) Int. Cl.: A61B 1/32

(54) **A disposable speculum**
Spekulum für einmaligen Gebrauch
Speculum jetable

(30) Priority: 05.07.1989 IT 2895089 U
(43) Date of publication of application: 09.01.1991
(73) Proprietor: TECHNO TRANSFER S.r.l., 43100 Parma (IT)
(72) Inventor: Zurli, Guido Vittorio, I-43100 Parma (IT)
(74) Representative: Lanzoni, Luciano

(56) References cited:
- CH-A- 337 611
- DE-A- 3 718 150
- US-A- 3 817 242
- US-A- 4 686 966

## Description

The present invention relates to a disposable vaginal speculum.

The prior art embraces reusable vaginal speculums fashioned in steel, which must be thoroughly sterilized after each use.

There are also disposable speculums, manufactured in rigid plastic, of which the jaws and handgrip are embodied as two angled members, one acute and one obtuse, pivoted together at their vertices; the system whereby the two members are locked following insertion into the vagina and released subsequently to enable withdrawal, relies on the action of a soft plastic pin of rounded rectangular section, rotatable in and positively retained by a relative slot. Such a speculum is disclosed in DE 3 718 150.

The prior art betrays certain drawbacks, namely:
- in the case of the reusable speculum, the need for a thorough, hence costly sterilization every time the instrument is used, involving loss of time and a requirement for the appropriate equipment;
- in the case of the designs of disposable speculum in current use, the fact that the locking system is not automatic but requires manipulation of the pin on the part of the practitioner; in short, both hands are required to effect the locking operation.

The object of the present invention is to overcome the drawbacks mentioned above, and in particular to allow of locking the jaws of a vaginal speculum in the open position automatically following insertion of the instrument.

The stated object is fully realized in a disposable vaginal speculum as characterized in the appended claims, which comprises two structures fashioned in rigid plastic, one of which angled acutely and the other obtusely, pivoted together at their vertices and constituting the jaws and the handgrip of the instrument; the external half of the grip exhibits a slot through which to insert a pin fixed by a double stop ring to the half of the grip nearest the vagina.

The instrument is capable of adopting two operative positions, namely with the jaws closed and handgrip spread, enabling insertion into or withdrawal from the vagina, and with jaws spread and grip closed to enable inspection of the vagina. According to the invention, the pin is locked in a simple vertical movement brought about internally of a triangular slot when, following insertion of the instrument into the vagina with the jaws closed, the jaws are spread apart by squeezing the grip with one hand.

The invention will now be described in detail, by way of example, with the aid of the accompanying drawings, in which:
- fig 1 shows the speculum in perspective, with jaws closed;
- fig 2 shows the speculum in perspective, with the jaws spread apart.

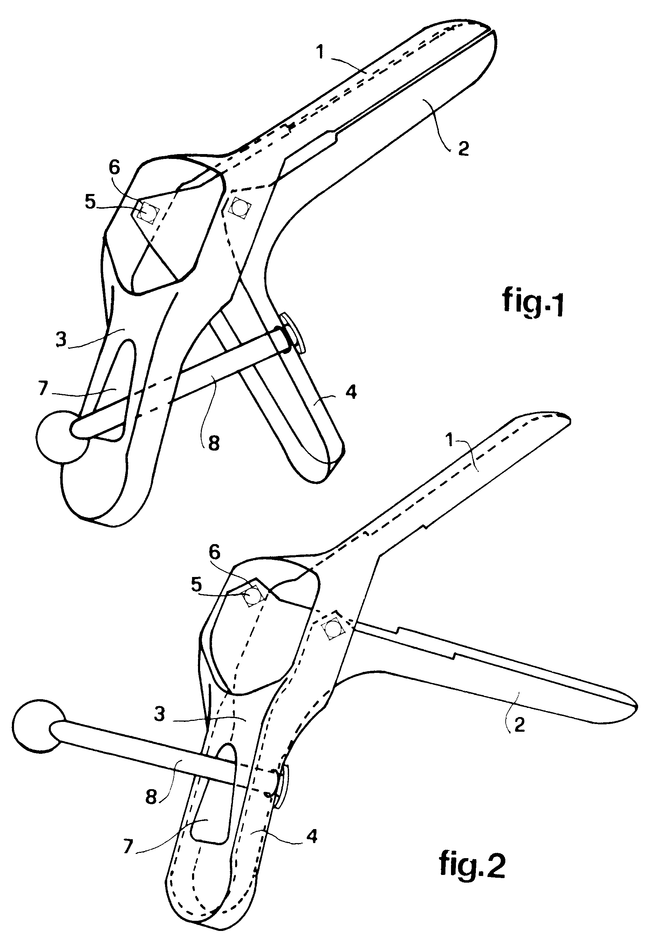

With reference to fig 1 of the drawings, 1 and 2 denote the jaws of the speculum, which are disposed respectively at an obtuse angle and an acute angle to corresponding halves 3 and 4 of the handgrip, forming two structures; the vertex of the acutely angled structure affords two projections 5 anchored in relative seatings 6 afforded by the outermost, obtusely angled structure.

7 denotes a triangular slot formed in one half 3 of the grip, which serves to accommodate a cylindrical pin 8 attached to the remaining half 4 by way of two stop rings and afforded freedom of movement in a vertical direction, within the slot 7.

Operation of the speculum will now be described.

The speculum is inserted into the vagina in the position of fig 1, whereupon by applying pressure to the two halves 3 and 4 of the grip, the jaws 1 and 2 are caused to spread apart to the point of locking automatically, as the result of the pin 8 wedging in the narrow top part of the triangular slot 7 by which it is accommodated.

To release and withdraw the speculum, it suffices simply to push the pin 8 toward the bottom of the slot 7 and thus enable the relative jaw 2 to close against its counterpart 1.

Accordingly, the jaws of the speculum can be both locked and released using one hand only.

## Claims

1. A disposable vaginal speculum, comprising two rigid plastic structures, one angled acutely and one obtusely, pivoted together at their vertices and constituting the jaws (1, 2) and handgrip (3, 4) of the instrument, the external half of the grip (3) exhibiting a slot (7) through which to insert a pin (8) fixed by a double stop ring to the half of the inner grip (4) that is nearest the vagina in operation, and capable of adopting two operative positions:
- with jaws closed and handgrip spread, to enable insertion into or withdrawal from the vagina;
- with jaws spread and handgrip closed, to enable inspection of the vagina,
characterized
in that the slot (7) has triangular shape and in that the pin (8) which is inserted in said slot and the jaws are arranged in such a way that said pin (8) is caused to lock by means of the simple vertical movement brought about internally of said slot (7) when, following insertion of the instrument into the vagina with jaws (1, 2) closed, the jaws (1, 2) are made to spread apart by simple squeezing of the grip (3, 4).

2. A disposable vaginal speculum as in claim 1, wherein the jaws (1, 2) are constructed in such a way that they are released and brought together simply by vertical movement of the pin (8) toward the wider end of the triangular slot (7).

3. A disposable vaginal speculum as in preceding claims, wherein the pin (8) is cylindrical.

## Patentansprüche

1. Ein Spekulum für einmaligen Gebrauch, das zwei starre Kunststoffstrukturen aufweist, von denen eine scharf und eine stumpf abgewinkelt ist, die an ihren Spitzen zusammen schwenkbar sind und die die Klemmen (1, 2) und den Handgriff (3, 4) des Instruments bilden; die äußere Hälfte des Griffes (3) weist einen Schlitz (7) auf, durch den ein Stift (8) eingeführt wird, welcher durch einen doppelten Stoppring an der Hälfte des inneren Handgriffes (4) befestigt ist, der bei einem Eingriff der Vagina näher ist und zwei Arbeitspositionen einnehmen kann:
- mit geschlossenen Klemmen und gespreiztem Handgriff zum Einführen in oder zur Entnahme aus der Vagina;
- mit gespreizten Klemmen und geschlossenem Handgriff zur Untersuchung der Vagina;
dadurch gekennzeichnet,
daß der Schlitz (7) eine dreieckige Form aufweist und dadurch, daß der Stift (8), der in den genannten Schlitz eingeführt wird, und die Klemmen derart angeordnet sind, daß der besagte Stift (8) durch eine einfache vertikale Bewegung in den genannten Schlitz (7) einrastet, nach der Einführung des Instruments in die Vagina mit geschlossenen Klemmen (1, 2) spreizen sich die Klemmen (1, 2) durch einfaches Zusammendrücken des Handgriffs (3, 4).

2. Ein Spekulum für den einmaligen Gebrauch gemäß dem Anspruch 1, wobei die Klemmen (1, 2) derart konstruiert sind, daß sie einfach durch vertikale Bewegung des Stiftes (8) in Richtung auf das weitere Ende des dreieckigen Schlitzes (7) gelöst und zusammengebracht werden.

3. Ein Spekulum für den einmaligen Gebrauch gemäß den vorausgehenden Ansprüchen, wobei der Stift (8) zylindrisch ist.

## Revendications

1. Un spéculum vaginal jetable, qui comprend deux structures en plastique rigides, l'une à angle aigu et l'autre à angle obtus, montées ensemble sur un pivot à leurs sommets et constituant les mâchoires (1, 2) et la prise (3, 4) de l'instrument, la moitié extérieure de la prise (3) présentant une fente (7) à travers laquelle on peut introduire une cheville (8) fixée par un double anneau d'arrêt à la moitié de la prise intérieure (4) qui est la plus proche du vagin durant l'opération et qui peut prendre deux positions opérationnelles:
- avec les mâchoires fermées et la prise écartée, pour permettre l'introduction dans le vagin ou le retrait au-dehors de celui-ci;
- avec les mâchoires écartées et les prises fermées pour permettre l'inspection du vagin,
caractérisé
en ce fait que la fente (7) a une forme triangulaire et que la cheville (8) qui est introduite dans ladite fente et les mâchoires sont disposées de telle sorte que ladite cheville (8) est poussée à se bloquer grâce au simple mouvement vertical fait à l'intérieur de ladite fente (7) quand, à la suite de l'introduction de l'instrument dans le vagin avec les mâchoires (1, 2) fermées, on fait s'écarter les mâchoires (1, 2) en exerçant simplement une pression sur la prise (3, 4).

2. Un spéculum vaginal jetable comme celui de la revendication 1, dans lequel les mâchoires (1, 2) sont fabriquées de telle sorte qu'elles soient débloquées et réunies simplement par le mouvement vertical de la cheville (8) vers l'extrémité la plus large de la fente triangulaire (7).

3. Un spéculum vaginal jetable comme dans les revendications précédentes, dans lequel la cheville (8) est cylindrique.
